# FASCICULE DE BREVET EUROPEEN

(11) **EP 0 527 204 B1**
(45) Date de publication et mention de la délivrance du brevet: **10.08.1994**
(21) Numéro de dépôt: 91909781.6
(22) Date de dépôt: 03.05.1991
(51) Int. Cl.: A61B 17/60

(54) **DISPOSITIF DE FIXATION D'EXTREMITE DE BROCHE DISTALE A USAGE CHIRURGICAL**
AM AUSSERENDE EINES CHIRURGISCHEN NAGELS ZU BEFESTIGENDE VORRICHTUNG
DEVICE FOR SECURING THE DISTAL END OF A SURGICAL PIN

(30) Priorité: 04.05.1990 FR 9005635
(43) Date de publication de la demande: 17.02.1993
(73) Titulaire: AGHION, Michael, F-92400 Courbevoie (FR)
(72) Inventeur: AGHION, Michael, F-92400 Courbevoie (FR)
(74) Mandataire: Dawidowicz, Armand
(86) Numéro de dépôt international: FR9100367
(87) Numéro de publication internationale: WO9116860

(56) Documents cités:
- FR-A- 1 345 527
- FR-A- 2 633 822
- US-A- 2 398 915

## Description

L'invention concerne un élément de fixation distale universel, en particulier pour la protection externe de l'extrémité d'une broche distale adaptable en particulier au doigt de la main mais non exclusivement pour celle-ci.

Les broches distales utilisées couramment en chirurgie de la main pour la consolidation des articulations ont des diamètres allant de 0,8mm à 3mm en progression de 0,2mm à 0,5mm. Il existe pour protéger l'extrémité de cette broche, dans la partie externe, un élément de fixation distale en forme de bille en matière plastique résistant au passage en autoclave. Cette bille est percée de pôle en pôle d'un trou permettant le passage de broches distales de diamètres voisins. Par exemple, un élément de fixation distale dont l'alésage du trou sera de 1,2mm de diamètre acceptera les broches de 0,8mm, 1,00mm, 1,2mm. Pour les diamètres encore plus importants, le diamètre extérieur de la bille sera plus gros. Tous ces éléments de fixation distale existants sont percés dans le plan de l'équateur d'un trou taraudé dans lequel l'action de vissage d'un tournevis sur une vis sans tête vient faire une pression ponctuelle sur la broche insérée dans l'élément, de sorte que cet élément se trouve ainsi immobilisé en rotation et en translation sur la broche, évitant ainsi à celle-ci une migration dans la main, ou dans tout autre partie du corps où cette broche pourrait être implantée.

L'élément de fixation distale facilite par sa forme esthétique l'exécution des soins et pansements, il évite ainsi au chirurgien d'exécuter un pliage à 90° de la broche distale comme cela se pratiquait avant la création de l'élément de fixation distale.

Pour permettre l'utilisation d'un élément de fixation de ce type pour l'ensemble des diamètres des broches existantes, on a proposé (FR-A-2.633.822) de munir l'élément de fixation de deux alésages orthogonaux au croisement desquels débouche le trou taraudé de la vis de serrage. Ce dispositif connu est d'utilisation malaisée pour le chirurgien qui doit choisir l'alésage correspondant à la broche qu'il a mise en place. En outre, l'alésage non utilisé constitue un réceptacle de produits organiques et organismes qui sont dangereux pour la plaie adjacente.

La présente invention vise à supprimer ces inconvénients du dispositif de fixation connu selon FR-A-2.633.822, grâce à un nouveau dispositif s'adaptant sans hésitation à tous les diamètres des broches existantes et qui ne présente pas d'alésage ouvert après sa mise en place.

A cet effet, l'invention a pour objet un élément de fixation distale universel pour broches chirurgicales de tous les diamètres utilisés, du type comportant une sphère de protection munie d'un alésage pour une broche et une vis de serrage logée dans un trou taraudé qui débouche dans ledit alésage, caractérisé en ce qu'il comporte un insert élastiquement déformable par l'intermédiaire de ladite vis de serrage et venant en contact avec au moins une génératrice de la broche à protéger, l'insert déformable étant en forme de croissant dont la longueur de l'arc concave est approximativement égale à la longueur de la circonférence de la plus petite broche admissible, le rayon avant déformation correspondant au rayon du plus gros diamètre de broche admissible.

L'élément de fixation selon l'invention permet ainsi une adaptation simple et sûre à tous les diamètres de broche, avec un seul alésage pour les différentes broches. L'élasticité de l'insert le fait revenir en position de libération par desserrage de la vis.

Selon une forme de réalisation préférée de l'invention, les deux extrémités de l'insert déformable en forme de croissant sont superposées dans la région d'action de la vis de serrage.

Avantageusement, ledit alésage a une section supérieure à celle de la broche de plus grande section, l'alésage traversant étant formé par une portion d'alésage à section circulaire de section correspondant à celle de la broche de plus grand diamètre auquel est raccordée une portion d'un alésage d'axe parallèle dont la section correspond à celle de la broche de plus petit diamètre.

L'invention sera bien comprise à la lecture de la description suivante faite en se référant au dessin annexé dans lequel :

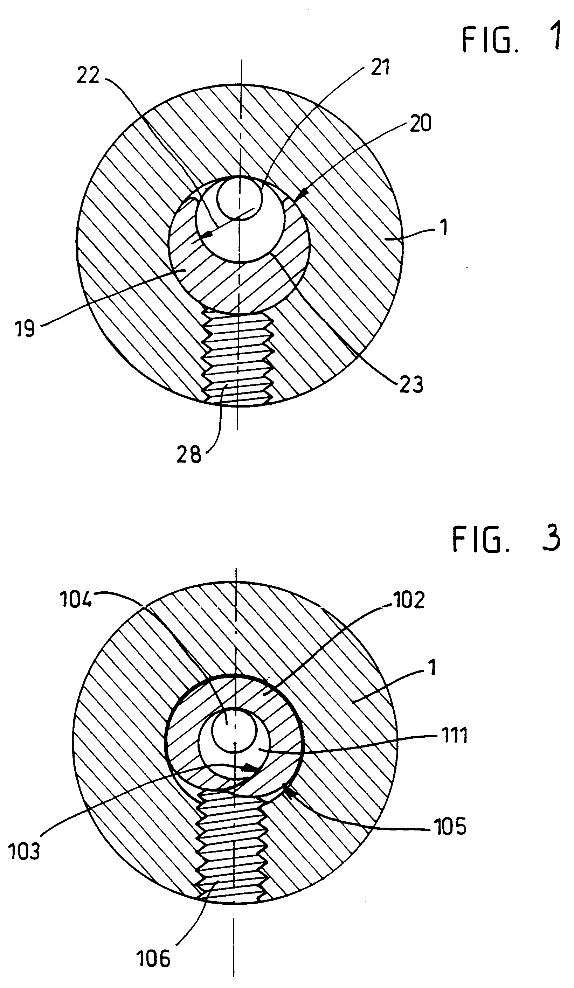

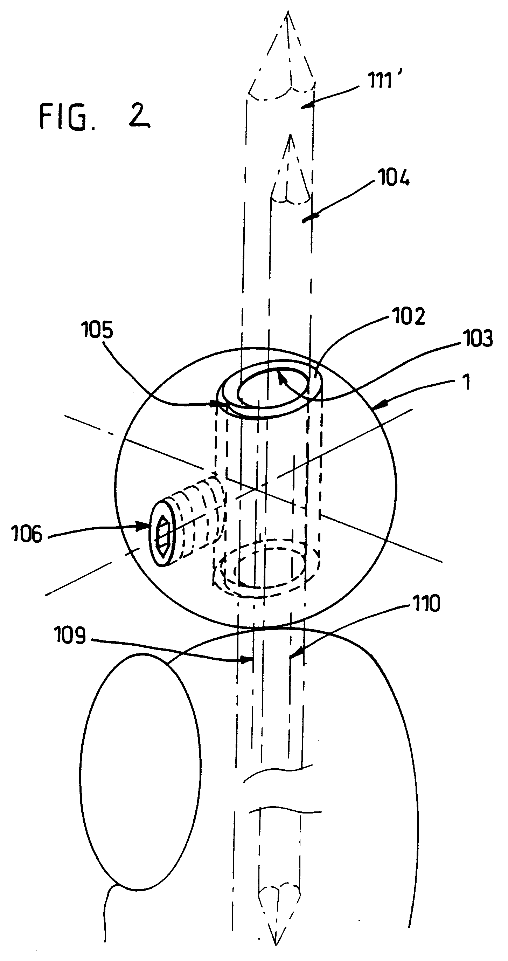

La figure 1 est une vue schématique en coupe diamétrale d'un dispositif de fixation selon un exemple de réalisation de l'invention; la figure 2 est une vue schématique en perspective d'un dispositif de fixation selon une variante de l'invention, et la figure 3 est une vue en coupe diamétrale à angle droit par rapport à la figure 2; la figure 4 est analogue à la figure 3, pour une variante; la figure 5 est analogue à la figure 1, pour une autre variante de l'invention, et la figure 6 est analogue aux figures 3 et 4, pour la variante de la figure 5.

Dans la forme de réalisation de la figure 1, un alésage unique 20 de section circulaire correspondant à un diamètre supérieur au diamètre maximum de broche admissible reçoit un insert déformable 19 qui, sous l'action de la vis 28, viendra envelopper l'extrémité de la broche à protéger. Cet insert déformable 19, en forme de croissant, pourra être obtenu par filage ou extrusion. La longueur de l'arc concave 23 de ce croissant sera égale approximativement à la longueur de la circonférence de la plus petite broche admissible 21. Son rayon 22 avant déformation correspondra au rayon engendré par le plus gros diamètre de broche admissible dans la sphère 1. Par l'action de poussée donnée par la vis 28, le glissement des génératrices de l'insert déformable 19 en forme de croissant le long des génératrices du trou 20 permet l'enveloppement de toutes les génératrices de l'extrémité de la broche distale à protéger, faisant ainsi participer un très grand nombre de génératrices à l'immobilisation de la sphère sur la broche distale.

Dans la forme de réalisation préférée des figures 2 et 3, on assure un meilleur centrage de la broche en faisant coïncider un diamètre 109 de la sphère 1 et l'axe 110 de la broche à protéger grâce au glissement de la face interne commençant à l'extrémité biseautée d'un ruban 102 d'épaisseur constante en forme de croissant, sur la face externe de l'autre extrémité biseautée du même ruban 102, dans un logement 111 de la sphère 1.

Le recouvrement spiralé du ruban 102, c'est-à-dire face interne 103 du ruban 102 contre face externe 105, et le glissement relatif de ces deux faces l'une contre l'autre sont réalisés par la rotation de la vis 106 qui, en entraînant la face externe 105 du ruban 102, force celui-ci à réduire la longueur de la circonférence interne d'origine. La vis de serrage 106 est munie d'un filetage dont le rapport pas/diamètre est de 8/100 contrairement au filetage des vis couramment utilisées qui est de 6/100. La cessation de l'action de serrage de la vis autorise, comme dans un ressort spirale, le retour du ruban dans sa forme d'origine, grâce à la propriété de mémoire de son matériau.

Le dispositif permet un serrage centré sur des broches 104 de petit diamètre aussi bien que sur des broches 111′ de grand diamètre.

## Revendications

1. Elément de fixation distale universel pour broches chirurgicales de tous les diamètres utilisés, du type comportant une sphère de protection munie d'un alésage pour une broche et une vis de serrage logée dans un trou taraudé qui débouche dans ledit alésage,
caractérisé en ce qu'il comporte un insert (17, 19, 102) élastiquement déformable par l'intermédiaire de ladite vis de serrage (28, 106) et venant en contact avec au moins une génératrice de la broche (14, 15, 101, 104) à protéger, ledit alésage (2, 3) ayant une section supérieure à celle de la broche de plus grande section (15, 111′), l'insert déformable (19, 102) étant en forme de croissant dont la longueur de l'arc concave est approximativement égale à la longueur de la circonférence de la plus petite broche admissible (21), le rayon avant déformation correspondant au rayon du plus gros diamètre de broche (23) admissible.

2. Elément de fixation selon la revendication 1,
caractérisé en ce que les extrémités de l'insert déformable (102) en forme de croissant sont superposées dans la région d'action de la vis de serrage (106).

3. Elément de fixation selon l'une des revendications 1 et 2,
caractérisé en ce que l'alésage traversant est formé par une portion d'alésage (2) à section circulaire de section correspondante à celle de la broche de plus grand diamètre (15) auquel est raccordée une portion d'un alésage (3) d'axe parallèle dont la section correspond à celle de la broche (14) de plus petit diamètre.

## Patentansprüche

1. Am äußeren Ende von chirurgischen Nägeln in allen benutzten Durchmessern zu befestigendes Universalelement, bestehend aus einer Schutzkugel mit einer Bohrung für einen Nagel und einer Klemmschraube, die in einem in die genannte Bohrung einmündenden Gewindeloch angeordnet ist, **dadurch gekennzeichnet,** daß es einen Einsatz (17, 19, 102) umfaßt, der durch die Klemmschraube (28, 106) elastisch verformbar ist und der mit mindestens einer Erzeugenden des zu schützenden Nagels (14, 15, 101, 104) in Berührung kommt, wobei die Bohrung (2, 3) einen größeren Querschnitt als der Nagel mit dem größten Querschnitt (15, 111′) besitzt und wobei der verformbare Einsatz (19, 102) eine Sichelform aufweist, deren konkaver Bogen eine Länge hat, die annähernd gleich der Länge des Kreisumfangs des kleinsten zulässigen Nagels (21) ist, während der Radius vor der Verformung dem Radius des größten zulässigen Durchmessers des Nagels (23) entspricht.

2. Befestigungselement nach Anspruch 1, **dadurch gekennzeichnet,** daß sich die Enden des verformbaren sichelförmigen Einsatzes (102) im Wirkungsbereich der Klemmschraube (106) überlagern.

3. Befestigungselement nach einem der Ansprüche 1 oder 2 , **dadurch gekennzeichnet**, daß die durchgehende Bohrung durch einen Bohrungsabschnitt (2) mit kreisförmigem Querschnitt gebildet wird, dessen Querschnitt dem Querschnitt des Nagels mit dem größten Durchmesser (15) entspricht und an den sich ein Bohrungsabschnitt (3) mit paralleler Achse anschließt, dessen Querschnitt dem Querschnitt des Nagels (14) mit dem kleinsten Durchmesser entspricht.

## Claims

1. A universal device for securing the distal end of surgical pins of all the diameters used, of the type comprising a protective sphere provided with a bore for a pin and a securing screw housed in a threaded hole which opens into the said bore, characterised in that it comprises an insert (17, 19, 102) which can be elastically deformed by means of the said securing screw (28, 106) and coming into contact with at least one generator of the pin (14, 15, 101, 104) to be protected, the said bore (2, 3) having a cross-section greater than that of the pin with the largest cross-section (15, 111′), the deformable insert (19, 102) being in the form of a crescent of which the length of the concave arc is approximately equal to the length of the circumference of the smallest permissible pin (21), the radius prior to deformation corresponding to the radius of the largest permissible diameter of pin (23).

2. A securing device according to claim 1, characterised in that the ends of the deformable crescent-shaped insert (102) are overlapped in the region of operation of the securing screw (106).

3. A securing device according to one of claims 1 and 2, characterised in that the through bore is formed by a portion of a bore (2) with a circular cross-section, the cross-section corresponding to that of the largest diameter pin (15), to which bore is connected a portion of a bore (3) having a parallel axis of which the cross-section corresponds to that of the smallest diameter pin (14).
